(19) Europäisches Patentamt
European Patent Office
Office européen des brevets

(11) **EP 2 005 165 B1**

(12) **EUROPEAN PATENT SPECIFICATION**

(45) Date of publication and mention
of the grant of the patent:
**24.10.2012 Bulletin 2012/43**

(51) Int Cl.:
***G01N 33/50*** *(2006.01)*  ***A61K 39/00*** *(2006.01)*

(21) Application number: **07734937.1**

(86) International application number:
**PCT/IB2007/001855**

(22) Date of filing: **21.03.2007**

(87) International publication number:
**WO 2007/122512 (01.11.2007 Gazette 2007/44)**

(54) **SEPARATION OF CONJUGATED AND UNCONJUGATED COMPONENTS**

TRENNUNG KONJUGIERTER UND NICHTKONJUGIERTER KOMPONENTEN

SÉPARATION DE COMPOSÉS CONJUGUÉS ET NON CONJUGUÉS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.03.2006 GB 0605757**

(43) Date of publication of application:
**24.12.2008 Bulletin 2008/52**

(60) Divisional application:
**11160129.0 / 2 363 709**

(73) Proprietor: **Novartis Vaccines and Diagnostics S.r.l.**
**53100 Siena (SI) (IT)**

(72) Inventors:
• **BERTI, Francesco**
**53100 Siena (IT)**
• **GALLETTI, Bruno**
**53100 Siena (IT)**
• **PARENTE, Pierino**
**53100 Siena (IT)**

• **COSTANTINO, Paolo**
**53100 Siena (IT)**

(74) Representative: **Marshall, Cameron John et al**
**Carpmaels & Ransford**
**One Southampton Row**
**London**
**WC1B 5HA (GB)**

(56) References cited:
**WO-A-2005/090985     US-B1- 6 248 570**

• **LEI Q P ET AL: "Quantification of free polysaccharide in meningococcal polysaccharide-diphtheria toxoid conjugate vaccines" PHYSICO-CHEMICAL PROCEDURES FOR THE CHARACTERIZATION OF VACCINES, KARGER, BASEL, CH, vol. 103, 2000, pages 259-264, XP002989234**

Remarks:
The file contains technical information submitted after the application was filed and not included in this specification

EP 2 005 165 B1

**Description**

**TECHNICAL FIELD**

This invention concerns the analysis and quality control of vaccines that include saccharides (*e.g.* bacterial capsular saccharides), and especially those where the saccharides are conjugated to a carrier. In particular, the invention is useful for the analysis and quality control of conjugate vaccines comprising a conjugate of a saccharide containing a sialic acid residue, *e.g.* a bacterial capsular saccharide from *Streptococcus agalactiae* (also known as group B streptococcus (GBS)).

**BACKGROUND OF THE INVENTION**

Immunogens comprising capsular saccharide antigens conjugated to carrier proteins are well known in the art. Conjugation converts T-independent antigens into T-dependent antigens, thereby enhancing memory responses and allowing protective immunity to develop, and the prototype conjugate vaccine was for *Haemophilus influenzae* type b (Hib) [*e.g.* see chapter 14 of ref 1]. Since the Hib vaccine, conjugated saccharide vaccines for protecting against *Neisseria meningitidis* (meningococcus) and against *Streptococcus pneumoniae* (pneumococcus) have been developed. Other organisms where conjugate vaccines are of interest are *Streptococcus agalactiae* (GBS) [2], *Pseudomonas aeruginosa* [3] and *Staphylococcus aureus* [4].

Conjugate vaccines for *N.meningitidis* serogroup C have been approved for human use, and include Menjugate™ [5], Meningitec™ and NeisVac-C™. Mixtures of conjugates from each of serogroups A, C, W135 and Y have been reported [*e.g.* refs. 6-9], including the Menactra™ product. Other mixtures of conjugated antigens include: (i) meningococcal A/C mixtures [10,11]; (ii) the PrevNar™ product [12] containing seven pneumococcal conjugates; (iii) mixed meningococcal and Hib conjugates [13,14]; and (iv) combined meningococcal, pneumococcal and Hib conjugates [15].

Problems when dealing with conjugate vaccines include stability and batch-to-batch consistency. In Hib vaccines, for instance, catalytic depolymerisation of the saccharide has been reported [16], and conjugates of the serogroup A meningococcus capsule are readily hydrolysed [17]. Instability of conjugates undesirably leads to a reduction in effective dose of immunogenic conjugate over time, variation between batches, and increased levels of uncharacterised breakdown products. References 18 & 19 discuss issues concerning stability testing of Hib conjugate vaccines.

Consequently, hydrolysis of the glycoconjugates to free (*i.e.* unconjugated) saccharide needs to be monitored in the formulated vaccines alone or when in combination with other vaccines. This analysis typically requires separation of any unconjugated saccharide from the conjugate followed by quantitative saccharide analysis. Known methods of separation include ultrafiltration, hydrophobic chromatography and selective precipitation [20].

It is an object of the invention to provide modifications and improvements in the quality control of conjugate vaccines for assessing their stability and integrity. In particular, it is an object to provide improved separation techniques prior to quantitative analysis.

**DISCLOSURE OF THE INVENTION**

The inventors have discovered that conjugated saccharide may be selectively precipitated from unconjugated saccharide with a basic reagent under basic conditions. Under these conditions, the unconjugated saccharide remains substantially in the supernatant while the conjugated saccharide is substantially fully precipitated, thereby allowing rapid and quantitative separation of these saccharides which may be exploited in analytical methods for quantifying unconjugated saccharide. Reference 20 describes the selective precipitation of free unconjugated saccharide from conjugated saccharide in meningococcal polysaccharide-diphtheria toxoid conjugate vaccines using deoxycholate/HCl under acidic conditions. However, it has been discovered that this approach is limited, since bacterial capsular saccharides containing sialic acid residues, especially terminal sialic acid residues (*e.g. Streptococcus agalactiae*) are liable to hydrolysis under these conditions.

The inventors have also discovered that conjugated saccharide may be selectively precipitated from unconjugated carrier using the same reagent and conditions. Similarly, the unconjugated carrier remains substantially in the supernatant while the conjugated saccharide is substantially fully precipitated, thereby allowing rapid and quantitative separation of these components which may be exploited in analytical methods for quantifying unconjugated carrier (and therefore may be used for quantifying unconjugated saccharide).

The invention overcomes the deficiencies in the prior art and provides a rapid and quantitative technique for separation of unconjugated and conjugated components. The invention is applicable to a range of conjugate vaccines, including vaccines comprising bacterial capsular saccharides containing a sialic acid residue and particularly vaccines comprising *Streptococcus agalactiae* capsular saccharide.

According to a first aspect of the invention, a basic reagent is employed under basic conditions to separate conjugated saccharide from an unconjugated component, as set out in the appended claims. Thus, the invention provides a method of separating a conjugated saccharide component in a sample from an unconjugated component in the sample, comprising the step of contacting the sample with a basic reagent under basic conditions to selectively precipitate the conjugated saccharide component from the sample, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11. The invention also provides the use of a basic reagent under basic conditions for selectively precipitating a conjugated saccharide component in a sample from an unconjugated component in the sample, thereby separating the conjugated saccharide component from the unconjugated component, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11.

In a second aspect, the invention provides a method of preparing a sample comprising a conjugated saccharide component and an unconjugated component for analysis of its degree of unconjugation, comprising the step of contacting the sample with a basic reagent under basic conditions to selectively precipitate the conjugated saccharide component from the sample and thereby to obtain a supernatant comprising the separated unconjugated component, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11. The invention also provides a method of analysing the degree of unconjugation of a sample comprising a conjugated saccharide component and an unconjugated component, comprising the steps of (i) contacting the sample with a basic reagent under basic conditions to selectively precipitate the conjugated saccharide component from the sample and thereby to obtain a supernatant comprising the separated unconjugated component and (ii) analysing the supernatant's content to give the unconjugated content of the sample, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11.

In a third aspect, the invention provides a method of releasing a vaccine for distribution to and/or use by physicians, comprising the steps of: (a) manufacturing a vaccine comprising a conjugated saccharide; (b) analysing the vaccine's degree of unconjugation by a method of analysis of the invention; and, if the results from step (b) indicate a degree of unconjugation acceptable for clinical use, (c) releasing the vaccine for use by physicians. Step (b) may involve assessment of minimum saccharide concentration (*e.g.* between 1-20 $\mu$g of total saccharide), assessment of unconjugated:conjugated saccharide ratio (*e.g.* $\leq$ 20% of the total saccharide by weight is unconjugated saccharide, preferably $\leq$ 10%, $\leq$ 5%, *etc*). Step (b) may be performed on a packaged vaccine, or may be performed on a bulk vaccine prior to packaging. Where the vaccine is a combination vaccine, step (b) may be performed in respect of an individual type of saccharide or of all types of saccharide.

### Basic reagent

The term "basic reagent" includes reagents capable of forming a basic solution (*i.e.* pH>7) on addition to water. The basic reagent can be a basic solution itself (*e.g.* a solution of a basic salt), a solid reagent (*e.g.* a basic salt), or a gas (*e.g.* NH$_3$). The basic reagent may comprise a single reagent, or a mixture of reagents, *e.g.* a mixture of basic solutions, or a mixture of solid reagents. Optionally, the basic reagent may also comprise a buffer, in order to form the appropriate pH conditions on addition of the basic reagent to the sample.

The basic reagent typically comprises a base, which can be in a solid, gaseous or aqueous state *etc.,* as appropriate. Preferred bases are lyotropic salts, *e.g.* sulphates, hydrogen phosphates, acetates, citrates or tartrates of ammonium, potassium or sodium. Particularly preferred lyotropic salts are sulphates and hydrogen phosphates of ammonium and potassium, *i.e.* K$_2$HPO$_4$, (NH$_4$)$_2$HPO$_4$, K$_2$SO$_4$ or (NH$_4$)$_2$SO$_4$. A particularly preferred lyotropic salt is K$_2$HPO$_4$

Basic solutions of basic salts are preferred basic reagents. Basic salt concentrations greater than 1M are preferred, with saturated solutions (*e.g.* of K$_2$HPO$_4$) being particularly preferred. When basic solutions are used, the volume of basic solution to sample is typically within 1:4 to 2:1, preferably within 1:3 to 1.67:1, more preferably about 1:1).

### Basic Conditions

The sample is contacted with the basic reagent under basic conditions, *i.e.* at a final pH of from pH 9 to 11, more preferably from pH 9.5 to 9.9, still more preferably about pH 9.7.

The basic conditions can be achieved by contacting the sample with the basic reagent, optionally in combination with a buffer, in amounts to obtain an appropriate concentration of the basic reagent, and optionally the buffer. The pH of the mixture after contacting will depend on the initial pHs of the sample, the basic reagent, and the optional buffer.

Where the basic reagent is an aqueous solution, the pH of the basic reagent before contacting with the sample is >7, preferably from pH 8 to 12, more preferably from pH 9 to 11, more preferably from pH 9.5 to 10.5, still more preferably from pH 10 to 10.3. Preferably, the pH of the basic reagent before contacting is greater than the pH of the sample before contacting. A preferred basic reagent is a saturated solution of $K_2HPO_4$ (pH 10.30). The pH of the sample is not critical to the invention, provided sufficient basic reagent, optionally in combination with a buffer, can be contacted with the sample to provide basic conditions of from pH 9 to 11. Preferably, however, the sample is about neutral or basic, *e.g.* having a pH >6. Conjugate vaccines will typically be buffered at about neutral pH, *e.g.* having a pH from 6 to 8, preferably about 7.2. In a preferred embodiment of the invention, the preferred basic conditions are provided in combination with the use of a basic reagent comprising a monohydrogen phosphate salt, particularly $K_2HPO_4$. Basic conditions from pH 9.5 to 9.9 in combination with the use of a basic reagent comprising $K_2HPO_4$ are especially preferred, as the inventors have discovered that this combination leads to particularly effective selective precipitation of *Streptococcus agalactiae* (GBS) conjugates.

*Component Types*

The unconjugated component is an unconjugated saccharide component or an unconjugated carrier component, as discussed in more detail below.

Therefore, in the methods of analysis of the invention, where the unconjugated component is an unconjugated saccharide component, step (ii) comprises analysing the supernatant's saccharide content to give the unconjugated saccharide content of the sample. Similarly, where the unconjugated component is an unconjugated carrier component, step (ii) comprises analysing the supernatant's carrier content to give the unconjugated carrier content of the sample. Optionally, the invention can comprise analysing both the supernatant's saccharide content and carrier content.

*Degree of Unconjugation*

A sample's degree of unconjugation can be assessed in several ways, but all involve, either directly or indirectly, analysing a sample's unconjugated saccharide and/or carrier content. It can be analysed absolutely, *e.g.* unconjugated saccharide and/or carrier content can be used directly as a measure of a sample's degree of unconjugation. Alternatively, it can be analysed relatively, *e.g.* the ratio of a sample's unconjugated saccharide content to its total (*i.e.* unconjugated and conjugated) saccharide content, and/or the ratio of a sample's unconjugated carrier content to its total (*i.e.* unconjugated and conjugated) carrier content, can be used as a measure of a sample's degree of unconjugation.

Typically, there is a relationship between the unconjugated saccharide content and the unconjugated carrier content of a sample, and the unconjugated saccharide content can be determined from the unconjugated carrier content of a sample, and *vice versa.* Thus, analysing the unconjugated saccharide content of a sample constitutes a method of analysing the unconjugated carrier content of a sample, and *vice versa.* For example, this relationship can be advantageously used, *e.g.* when analysing a conjugate vaccine comprising a single carrier but more than one type of saccharide. Instead of analysing the degree of unconjugation for each type of saccharide in the vaccine, the degree of unconjugation of the carrier can be analysed to give a degree of unconjugation for the vaccine as a whole, *i.e.* an average degree of unconjugation for all the types of saccharide.

When assessing a sample's degree of unconjugation, it is preferred that a sample's unconjugated saccharide and/or carrier content is analysed directly. However, a sample's degree of unconjugation may be analysed indirectly, *e.g.* by analysing a sample's conjugated saccharide and/or conjugated carrier content. The total saccharide content = unconjugated saccharide content + conjugated saccharide content and the total carrier content = unconjugated carrier content + conjugated carrier content, and these relationships may be exploited to calculate the unknown variable from the two known variables. Thus, analysing the conjugated saccharide content and total saccharide content indirectly constitutes a method of analysing the unconjugated saccharide content of a sample; and analysing the conjugated carrier content and total carrier content indirectly constitutes a method of analysing the unconjugated carrier content of a sample.

Direct analysis of the unconjugated saccharide and/or unconjugated carrier content is preferred as it involves analysis of the supernatant rather than the precipitate. Being a liquid, the supernatant is generally better suited for carrier and/or saccharide analytical techniques than the precipitate.

Preferably, a sample's degree of unconjugation is assessed by analysing (preferably directly) a sample's unconjugated saccharide content. In general, it is desirable to ensure that a vaccine includes <25% (e.g. <20%, <15%, <10% *etc.*) of each saccharide type in free form. High levels of free saccharide mean a lower immunogenic dose of conjugate.

The method of analysis preferably comprises the step of measuring the total saccharide content and/or the total carrier content of the sample. Typically, this step will involve a preparative step of dividing the sample, one portion of the same being analysed for unconjugated saccharide or carrier content, another portion being analysed for total saccharide or carrier content.

Alternatively, the unconjugated saccharide or carrier content may be compared against a known or theoretical total saccharide or carrier content the sample, or a standard saccharide or carrier content of the sample, as appropriate (*e.g.* the calculated amount of unconjugated saccharide or carrier present after a known period of time), instead of measuring the total saccharide or carrier content. In this embodiment, therefore, the method of analysis comprises the step of comparing the unconjugated saccharide or carrier content against a known or theoretical total saccharide or carrier content of the sample or a standard saccharide or carrier content of the sample.

### Methods of Saccharide Analysis

Methods of analysing the unconjugated or conjugated saccharide content typically require separation of the unconjugated saccharide from the conjugated saccharide. Consequently, the analytical methods of the invention typically comprise the steps of (i) an initial preparation step of contacting the sample with a basic reagent under basic conditions to precipitate the conjugated saccharide component selectively from the sample and thereby to obtain a supernatant comprising separated unconjugated saccharide, followed by (ii) analysing the supernatant's saccharide content to give the unconjugated saccharide content of the sample. The present invention also provides a method of preparing a sample for analysis by carrying out step (i). Optionally, after step (i), but prior to step (ii), the sample can be centrifuged and/or filtered.

### Calculation and Measurement

In general, samples can be analysed in several ways using the invention. Unconjugated saccharide content can be measured *e.g.* to check for incomplete conjugation, or to follow conjugate hydrolysis by monitoring increasing free saccharide over time. In addition, by measuring total (*i.e.* unconjugated and conjugated) saccharide content in a sample, the ratio of free saccharide to total saccharide can be assessed (if necessary in respect of each saccharide type), which can be used for regulatory or quality control purposes. In general, it is desirable to ensure that a vaccine includes <25% (*e.g.* <20%, <15%, <10% *etc.*) of each saccharide type in free form. High levels of free saccharides mean a lower immunogenic dose of conjugate.

As an alternative, or in addition, to measuring the unconjugated saccharide content, the conjugated saccharide content can also be measured *e.g.* to check for incomplete conjugation, or to follow conjugate hydrolysis by monitoring increasing free saccharide over time.

The method of analysis preferably comprises the step of measuring the total saccharide content of the sample. Typically, this step will involve a preparative step of dividing the sample, one portion of the same being analysed for unconjugated saccharide content, another portion being analysed for total saccharide content.

Alternatively, the unconjugated saccharide content may be compared against a known or theoretical total saccharide content of the sample or a standard saccharide content of the sample (*e.g.* the calculated amount of unconjugated saccharide present after a known period of time), instead of measuring the total saccharide content. In this embodiment, therefore, the method of analysis comprises the step of comparing the unconjugated saccharide content against a known or theoretical total saccharide content of the sample or a standard saccharide content of the sample.

### Separating Different Types of Saccharide

The conjugated saccharides in the sample typically comprise saccharide antigens conjugated to carrier proteins. Frequently, the saccharides derived from a particular pathogen comprise oligo- or polysaccharides having a distribution of lengths. The saccharides comprising this distribution are all considered to be of the same "type". In a combination vaccine, *e.g.* a combination vaccine comprising a mixture of meningococcal capsular saccharides of serogroups C, W135 and Y, the vaccine will comprise a saccharide "type" associated with each component immunogen, *e.g.* a saccharide type associated with serogroup C immunogens, a saccharide type associated with serogroup W135 immunogens and a saccharide type associated with serogroup Y immunogens.

The methods of the invention separate unconjugated and conjugated saccharides and do not generally distinguish between different saccharide types, thus separating unconjugated and conjugated saccharide regardless of saccharide type.

However, the measurement of the unconjugated saccharide content of the individual types of saccharide in a combination vaccine is nevertheless possible. Certain methods of quantitative glycoconjugate analysis discussed in more detail below allow the measurement of individual types of saccharide within combined glycoconjugate vaccines comprising more than one type of glycoconjugate immunogen, even where different saccharides share monosaccharide units. Consequently, the methods of the invention allow the analysis of the unconjugated saccharide content of a single or combined vaccine and, in respect of a combined vaccine comprising more than one type of glycoconjugate immunogen, for each individual type of saccharide or for all types of saccharide.

*Measuring the Saccharide Content of a Composition*

As mentioned above, in addition to measuring the saccharide content of the separated unconjugated saccharide, the method of analysis of the present invention may optionally include the step of measuring the total (*i.e.* unconjugated and conjugated) saccharide content of an individual type of saccharide or the content of all types of saccharide. The total saccharide content may be measured by measuring the saccharide content of the sample before separation. Thus, the present invention may require the measurement of the saccharide content of the separated unconjugated saccharide and the total saccharide of the sample before, after or at the same time as separation.

Methods for measuring the saccharide content of compositions are well known in the art and typical techniques include colorimetry analysis, and high performance anion exchange chromatography (HPAEC) in combination with pulsed amperometric detection (PAD).

Typically, the saccharide is treated in order to depolymerise the saccharides to give their constituent monosaccharides prior to analysing the monosaccharide content. Analysis of saccharide content can then proceed on the depolymerised mixture of released monosaccharides. Since the monosaccharide content is directly related to the content of saccharide in the composition to be analysed prior to depolymerisation, analysis of the saccharide content of depolymerised monosaccharides allows determination of the saccharide content of the composition.

Conditions for depolymerisation of saccharides to their constituent monosaccharides are known in the art and typically comprise acid or base hydrolysis.

For example, the serogroup C saccharide can be hydrolysed for total saccharide content analysis by treatment with 100 mM HCl at 80°C for 2 hours [21]. Acid hydrolysis using HCl may also be used for the Hib saccharide and typical treatment involves addition of TFA to a final concentration of 0.3M and heating at 100°C for 2 hours. Acid hydrolysis using trifluoroacetic acid (TFA) can be used for hydrolysis of all of serogroups C, W135 and Y, with a slightly lower incubation temperature being preferred for serogroup C to avoid degradation of its sialic acid (90°C rather than 100°C). A typical TFA treatment involves addition of TFA to a final concentration of 2M, followed by heating to 90-100°C for 90 minutes. Acid hydrolysis using TFA can also be used for hydrolysis of MenA polysaccharide and typical treatment involves addition of TFA to a final concentration of 2M and heating at 100°C for 2 hours. Acid hydrolysis using TFA can also be used for hydrolysis of Hib saccharide and typical treatment involves addition of TFA to a final concentration of 4M and heating at 100°C for 2 hours [22]. Base hydrolysis using NaOH can also be used for hydrolysis of Hib saccharide [22].

GBS saccharides can be depolymerised by hydrolysis in mild acid or by heating *etc*. A preferred acid hydrolysis is with 2M trifluoroacetic acid at 100°C for 2 hours [23]. Depolymerisation of the serotype III capsular saccharide by endo-β-galactosidase has been reported [24, 25, 26 and 27]. Ozonolysis of capsular polysaccharides from GBS serotypes II, III and VIII has also been used for depolymerisation [28].

After depolymerisation, saccharide hydrolysates may be dried *e.g.* using a vacuum drier.

After depolymerisation of a combination vaccine comprising more than one type of glycoconjugate immunogen, or the separated unconjugated saccharide from such a combined vaccine, the composition may contain mixed monosaccharides of different types. The quantities of these monosaccharides in the mixture are directly related to the quantities of saccharides in the original pre-hydrolysis composition, and so quantities of the starting saccharides can be determined by utilising the methods of the invention in the process for analysing the saccharide content of a composition disclosed in reference 29 and/or its priority application.

Progress of depolymerisation (*e.g.* to check for total hydrolysis to monosaccharides rather than partial hydrolysis to oligosaccharides) can be checked by measuring the degree of polymerisation (DP) in a mixture, using known techniques *e.g.* NMR, mass spectrometry, *etc*.

Once the saccharide has been depolymerised to monosaccharides, the monosaccharides are quantified. Consequently, the methods of analysis of the invention therefore typically comprise the step of quantifying the monosaccharides obtained from the depolymerisation step.

Methods for quantifying monosaccharides, including glucose *(e.g.* for *N.meningitidis* serogroup Y saccharide), galactose *(e.g.* for *N. meningitidis* serogroup W135 saccharide or a GBS saccharide), sialic acid *(e.g.* for *N.meningitidis* serogroup C saccharide or a GBS saccharide), ribitol *(e.g.* for Hib saccharide) and mannosamine-6-P *(e.g.* for *N.meningitidis* serogroup A saccharide) are well known in the art.

Methods of quantification may be direct or indirect *(e.g.* they may involve derivatisation of the monosaccharides followed by an analysis that correlates with original monosaccharide content). If necessary, methods may involve separation of the different monosaccharides from each other, followed by separate analysis, and in such a case the actual measurement of monosaccharide content could be the same in each case, with specificity arising from the separation. It is preferred, however, to use methods which can analyse the saccharides in each other's presence, such that they do not need to be separated from each other before analysis. In addition, methods may be used for conjugated saccharides in which, after deconjugation, the carrier and the saccharide need not be separated. One preferred method is anion chromatography, and in particular high performance anion exchange chromatography (HPAEC), in particular with pulsed amperometric detection (PAD) [30,31]. HPAEC-PAD systems are provided by Dionex™ Corporation (Sunnyvale, CA) *e.g.* the BioLC™ system, using a column such as PA1 [10 μm diameter polystyrene substrate 2% crosslinked with divinylbenzene, agglomerated with 500 nm MicroBead quaternary ammonium functionalized latex (5% crosslinked)] or PA10 [10 μm diameter ethylvinylbenzene substrate 55% crosslinked with divinylbenzene, agglomerated with 460 nm MicroBead difunctional quaternary ammonium ion (5% crosslinked)]. These systems can quantitatively analyse individual saccharides within mixtures without the need for derivatisation or pre-analysis separation. For saccharide analysis, it may be desired to filter other compounds before entry to the column, and Dionex™ produces pre-column traps and guards for this purpose *e.g.* an amino trap for removing amino acids, a borate trap, *etc.*

An alternative method for quantifying monosaccharides within a depolymerised mixture is nuclear magnetic resonance (NMR). For ease of use and for high sensitivity, however, the chromatographic methods of the invention are preferred.

A further method for quantifying monosaccharides is by colorimetry, *e.g.* as described in references 22; 32 and 94. However, it has been discovered that in the methods of the invention employing a basic reagent comprising ammonium sulphate are incompatible with colorimetry quantification. Preferably, therefore, where a colorimetric assay is used (*e.g.* for analysing saccharide content), the basic reagent does not comprise ammonium sulphate, and preferably does not comprise an ammonium salt.

Once the monosaccharide content has been determined, it is straightforward to calculate the quantity of saccharides in the original composition.

The process of the invention is typically destructive. Rather than perform the process on a complete composition, therefore, it is more typical to take a sample from a composition of interest and then perform the analysis on the sample.

### *Methods of Carrier Analysis*

Methods of analysing the unconjugated or conjugated carrier content typically require separation of the unconjugated carrier from the conjugated carrier. Consequently, the analytical methods of the invention typically comprise the steps of (i) an initial preparation step of contacting the sample with a basic reagent under basic conditions to precipitate the conjugated carrier component selectively from the sample and thereby to obtain a supernatant comprising separated unconjugated carrier, followed by (ii) analysing the supernatant's carrier content to give the unconjugated carrier content of the sample. The present invention also provides a method of preparing a sample for analysis by carrying out step (i). Optionally, after step (i), but prior to step (ii), the sample can be centrifuged and/or filtered.

### *Calculation and Measurement*

In general, samples can be analysed in several ways using the invention. Unconjugated carrier content can be measured *e.g.* to check for incomplete conjugation, or to follow conjugate hydrolysis by monitoring increasing free carrier over time. In addition, by measuring total (*i.e.* unconjugated and conjugated) carrier content in a sample, the ratio of free carrier to total carrier can be assessed (if necessary in respect of each saccharide type), which can be used for regulatory or quality control purposes. In general, it is desirable to ensure that a vaccine includes <25% (*e.g.* <20%, <15%, <10% *etc.*) of each carrier type in free form. High levels of free carriers mean a lower immunogenic dose of conjugate.

As an alternative, or in addition, to measuring the unconjugated carrier content, the conjugated content can also be measured *e.g.* to check for incomplete conjugation, or to follow conjugate hydrolysis by monitoring increasing free carrier over time. Since the total carrier content = unconjugated carrier content + conjugated carrier content, it is possible to calculate the carrier saccharide content by measuring the carrier saccharide content and the total carrier content. Thus, measuring the unconjugated carrier content and total carrier content constitutes a method of analysing the conjugated carrier content of a sample.

The method of analysis preferably comprises the step of measuring the total carrier content of the sample. Typically, this step will involve a preparative step of dividing the sample, one portion of the same being analysed for unconjugated carrier content, another portion being analysed for total carrier content.

Alternatively, the unconjugated carrier content may be compared against a known or theoretical total carrier content of the sample or a standard carrier content of the sample (*e.g.* the calculated amount of unconjugated carrier present after a known period of time), instead of measuring the total carrier content. In this embodiment, therefore, the method of analysis comprises the step of comparing the unconjugated carrier content against a known or theoretical total carrier content of the sample or a standard carrier content of the sample.

### *Measuring the Carrier Content of a Composition*

As mentioned above, in addition to measuring the carrier content of the separated Unconjugated carrier, the method of analysis of the present invention may optionally include the step of measuring the total (*i.e.* unconjugated and conjugated) carrier content. The total carrier content may be measured by measuring the carrier content of the sample before separation. Thus, the present invention may require the measurement of the carrier content of the separated unconjugated carrier and the total carrier of the sample before, after or at the same time as separation.

Methods for measuring the carrier content of compositions are well known in the art, *e.g.* as discussed in reference 33. Typical techniques for measuring the amount of protein carriers include the bicinchoninic acid (BCA) assay, the Bradford assay, the Lowry assay, UV absorbance assays, immunoassays (*e.g.* ELISA, RIA, *etc.),* and staining assays.

The process of the invention is typically destructive. Rather than perform the process on a complete composition, therefore, it is more typical to take a sample from a composition of interest and then perform the analysis on the sample.

### *Precipitation*

It is not critical to the invention whether any components which are not saccharide or carrier components of the sample are precipitated or left in the supernatant. Moreover, when the invention is concerned with only saccharide analysis, it is not critical to the invention whether any non-saccharide components of the sample are precipitated or left in the supernatant. Similarly, when the invention is concerned with only carrier analysis, it is not critical to the invention whether any non-carrier components of the sample are precipiated or left in the supernatant. Provided the unconjugated component of interest and the conjugated component end up separate from each other, then the unconjugated component is considered to have been separated from the conjugated component, regardless of which of the components is moved, which is present in the supernatant or which is present in the precipitate, and regardless whether either of the components is separated with other components.

Techniques for the separation of precipitate from supernatant are well known in the art, *e.g.* filtration or centrifugation.

### *Analysis of Non-Saccharide Components*

As well as analysing the content of carrier in a composition, the method of analysis may include analysis of other components or properties *e.g.* osmolality, pH, degree of polymerisation for individual saccharides or conjugates, saccharide content, aluminium content, detergent content, preservative content, *etc.*

### *Further Steps*

It may be desirable to remove at least some non-saccharide or non-carrier compounds from the sample prior to the step of separating conjugated and unconjugated components. For example, when the vaccine is formulated with aluminium-containing adjuvants, solid phase extraction may typically be applied to the supernatant, *e.g.* after centrifugation.

After analysis of the saccharide or carrier content, the invention may include the further step of determining a characteristic of a saccharide, *e.g.* its DP (typically an average DP), its molecular weight, its purity, *etc.*

After amperometric and spectroscopic detectors, the separated unconjugated or conjugated saccharide may be coupled into a mass spectrometer, *e.g.* FAB/MS or ESI/MS.

### *Preparation and Storage of Vaccines*

The invention provides a method for preparing a vaccine composition, comprising a step of analysis of a sample according to the invention, including a step of pH measurement, followed by a step of adjusting the pH of the composition to a desired value *e.g.* between 6 and 8, or about 7.

The invention provides a method for packaging a vaccine, comprising the steps of: (a) manufacturing a bulk vaccine containing a conjugated saccharide; (b) analysing the unconjugated saccharide content in the bulk vaccine by a method of analysis of the invention; (c) optionally, analysing the bulk vaccine for pH and/or other properties; and, if the results from step (b) and (c) indicate that the bulk vaccine is acceptable for clinical use, (d) preparing and packaging the vaccine for human use from the bulk. Step (c) may involve (see above) assessment of minimum saccharide concentration, assessment of unconjugated:conjugated saccharide ratio, *etc.* Step (d) may involve packaging into unit dose form or in multiple dose form *e.g.* into vials or into syringes. A typical human dose for injection has a volume of 0.5ml.

The invention also provides a method for packaging a vaccine, comprising the steps of: (a) manufacturing a bulk vaccine containing a conjugated saccharide; (b) analysing the Unconjugated carrier content in the bulk vaccine by a method of analysis of the invention; (c) optionally, analysing the bulk vaccine for pH and/or other properties; and, if the results from step (b) and (c) indicate that the bulk vaccine is acceptable for clinical use, (d) preparing and packaging the vaccine for human use from the bulk. Step (c) may involve (see above) assessment of minimum carrier concentration, assessment of unconjugated:conjugated carrier ratio, *etc.*

In these methods for packaging a vaccine, step (d) may involve packaging into unit dose form or in multiple dose form *e.g.* into vials or into syringes. A typical human dose for injection has a volume of 0.5ml.

Step (c) and/or (d) of the methods of packaging may be preceded by mixing the bulk vaccine with one or more further antigens *e.g.* with

- a capsular saccharide antigen from serogroup A of *N.meningitidis.*
- a capsular saccharide antigen from serogroup C of *N.meningitidis.*
- a capsular saccharide antigen from serogroup W135 of *N.meningitidis.*
- a capsular saccharide antigen from serogroup Y of *N.meningitidis.*
- a protein antigen from serogroup B of *N.meningitidis*
- preparations of *N.meningitidis* serogroup B microvesicles [34], 'native OMVs' [35], blebs or outer membrane vesicles *[e.g.* refs. 36 to 41 *etc.].*
- a saccharide antigen from *Haemophilus influenzae* type b
- an antigen from *Streptococcus pneumoniae,* such as polyvalent conjugated saccharide antigens *[e.g.* refs. 42 to 44].
- an antigen from hepatitis A virus, such as inactivated virus *[e.g.* 45, 46].
- an antigen from hepatitis B virus, such as the surface and/or core antigens [e.g. 46, 47].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 *[e.g.* refs. 48 & 49]. Cellular pertussis antigens may be used.
- a diphtheria antigen, such as a diphtheria toxoid *[e.g.* chapter 13 of ref. 1] *e.g.* the $CRM_{197}$ mutant *[e.g.* 50].
- a tetanus antigen, such as a tetanus toxoid *[e.g.* chapter 27 of ref. 1].
- polio antigen(s) [*e.g.* 51, 52], such as IPV.

Such antigens may be adsorbed to an aluminium salt adjuvant *(e.g.* a hydroxide or a phosphate). Any further saccharide antigens are preferably included as conjugates.

### *Samples*

The invention is particularly useful for analysing the unconjugated or conjugated saccharide content of a sample (*e.g.* a vaccine) or for preparing a sample for analysis of the unconjugated saccharide content of a sample (*e.g.* a vaccine). It is not essential to this embodiment that the invention contains any unconjugated or conjugated saccharide as the invention may be usefully employed to determine the presence or absence of unconjugated or conjugated saccharide. Moreover, a step of analysing the saccharide content of a sample of specimen which leads to a negative result, *i.e.* the absence of saccharide, is still a step of analysing the saccharide content of a sample or specimen.

However, it is preferred that the sample is suspected to contain (and preferably contains) conjugated saccharide or unconjugated saccharide. It is more preferred that the sample contain conjugated saccharide and is suspected to contain (and preferably contains) unconjugated saccharide.

The present invention is, however, more generally useful for separating conjugated saccharide from unconjugated saccharide in a sample (*e.g.* a vaccine). In this embodiment the sample preferably contains both conjugated saccharide and unconjugated saccharide.

The sample will generally be in aqueous solution.

As well as containing saccharides, samples to be analysed can include other materials. These may or may not be precipitated. Typically such components will not be precipitated.

The sample analyte may be a product to be tested prior to release (*e.g.* during manufacture or quality control testing), or may be a product to be tested after release (*e.g.* to assess stability, shelf-life, *etc.).*

Preferred samples are glycoconjugate vaccines, which may be single or combined (*e.g.* a combined glycoconjugated vaccine comprising more than one type of glycoconjugate immunogen). Particularly preferred samples are glycoconjugate vaccines comprising a conjugate comprising a saccharide containing a sialic acid residue, *e.g.* a bacterial capsular saccharide from *Streptococcus agalactiae* (group B streptococcus).

### *Conjugates*

The conjugated saccharides are covalently linked saccharide-carrier conjugates. Covalent conjugation is used to enhance immunogenicity of saccharides by converting them from T-independent antigens to T-dependent antigens, thus allowing priming for immunological memory. Conjugation is particularly useful for paediatric vaccines and is a well known technique [*e.g.* reviewed in refs. 53, to 62]. Saccharides may be linked to carriers (*e.g.* proteins) directly [63, 64], but a linker or spacer is generally used *e.g.* adipic acid, β-propionamido [65], nitrophenyl-ethylamine [66], haloacyl halides [67], glycosidic linkages [68], 6-aminocaproic acid [69], ADH [70], $C_4$ to $C_{12}$ moieties [71], *etc.*

Carrier Proteins in Conjugates

Typical carrier proteins in conjugates are bacterial toxins or toxoids, such as diphtheria toxoid or tetanus toxoid. The $CRM_{197}$ diphtheria toxin derivative [72-74] is the carrier protein in Menjugate™ and Meningitec™, whereas tetanus toxoid is used in NeisVac™. Diphtheria toxoid is used as the carrier in Menactra™. Other known carrier proteins include the *N.meningitidis* outer membrane protein [75], synthetic peptides [76,77], heat shock proteins [78,79], pertussis proteins [80,81], cytokines [82], lymphokines [82], hormones [82], growth factors [82], human serum albumin (preferably recombinant), artificial proteins comprising multiple human CD4+ T cell epitopes from various pathogen-derived antigens [83] (*e.g.* N19 [84]), protein D from *H.influenzae* [85,86], pneumococcal surface protein PspA [87], iron-uptake proteins [88], toxin A or B from *C.difficile.* [89], a GBS protein (particularly GBS67 or GBS80), *etc.* Compositions may use more than one carrier protein *e.g.* to reduce the risk of carrier suppression, and a single carrier protein might carry more than one saccharide antigen [90]. Conjugates generally have a saccharide:protein ratio (w/w) of between 1:5 (*i.e.* excess protein) and 5:1 (*i.e.* excess saccharide).

Saccharides in Conjugates

The conjugate saccharides may be polysaccharides (*e.g.* with a degree of polymerisation of >10, *e.g.* 20, 30, 40, 50, 60 or more) or oligosaccharides (*e.g.* with a degree of polymerisation of from 4 to 10). Oligosaccharides may be the result of depolymerisation and/or hydrolysis of a parent polysaccharide *e.g.* the analyte may be a saccharide-containing fragment of a larger saccharide. Preferred conjugate saccharides are capsular saccharides.

Even more preferred conjugate saccharides are bacterial capsular saccharides *e.g.* from *Neisseria meningitidis* (serogroups A, B, C, W135 or Y), *Streptococcus pneumoniae* (serotypes 4, 6B, 9V, 14, 18C, 19F, or 23F), *Streptococcus agalactiae* (types Ia, Ib, II, III, IV, V, VI, VII, or VIII), *Haemophilus influenzae* (typeable strains: a, b, c, d, e or f), *Pseudomonas aeruginosa, Staphylococcus aureus*, *etc.*

The *N.meningitidis* serogroup A capsule is a homopolymer of (a1→6)-linked N-acetyl-D-mannosamine-1-phosphate.

The *N.meningitidis* serogroup B capsule is a homopolymer of ($\alpha$ 2→8) linked sialic acids.

The *N.meningitidis* serogroup C capsular saccharide is a homopolymer of ($\alpha$ 2→9)-linked sialic acid (*N*-acetyl neuraminic acid, or 'NeuNAc'). Most serogroup C strains have O-acetyl groups at C-7 and/or C-8 of the sialic acid residues, but about 15% of clinical isolates lack these O-acetyl groups [91,92]. The acetylation does not seem to affect protective efficacy (*e.g.* unlike the Menjugate™ product, the NeisVac-C™ product uses a de-O-acetylated saccharide, but both vaccines are effective).

The *N.meningitidis* serogroup W135 saccharide is a polymer of sialic acid-galactose disaccharide units [→4)-D-Neu*p*5Ac(7/9OAc)-$\alpha$-(2→6)-D-Gal-$\alpha$-(1→]. Like the serogroup C saccharide, it has variable O-acetylation, but at sialic acid 7 and 9 positions [93].

The *N.meningitidis* serogroup Y saccharide is similar to the serogroup W135 saccharide, except that the disaccharide repeating unit includes glucose instead of galactose [→4)-D-Neu*p*5Ac(7/9OAc)-$\alpha$-(2→6)-D-Glc-$\alpha$-(1→]. Like the serogroup W135 saccharide, it has variable O-acetylation at sialic acid 7 and 9 positions [93].

The *H.influenzae* type b capsular saccharide is a polymer of ribose, ribitol, and phosphate ['PRP', (poly-3-$\beta$-D-ribose-(1, 1)-D-ribitol-5-phosphate)].

The *S.agalactiae* (GBS) capsular saccharide is covalently linked to the peptidoglycan backbone of GBS, and is distinct from the group B antigen, which is another saccharide that is attached to the peptidoglycan backbone.

The GBS capsular polysaccharides are chemically related, but are antigenically very different. All GBS capsular polysaccharides share the following trisaccharide core:

$\beta$-D-Glc*p*NAc(1→3)$\beta$-D-Gal*p*(1→4)$\beta$-D-Glc*p*

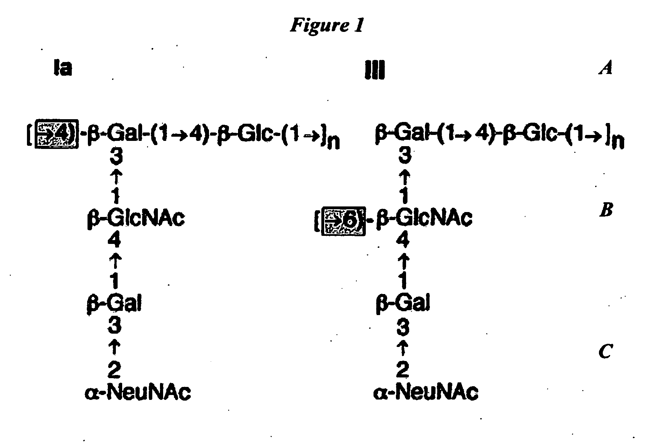

The various GBS serotypes differ by the way in which this core is modified. The difference between serotypes Ia and III, for instance, arises from the use of either the GlcNAc (Ia) or the Gal (III) in this core for linking consecutive trisaccharide cores (Figure 1). Serotypes Ia and Ib both have a [$\alpha$-D-Neu*p*NAc(2→3)$\beta$-D-Gal*p*-(1→] disaccharide linked to the GlcNAc in the core, but the linkage is either 1→4 (Ia) or 1→3 (Ib).

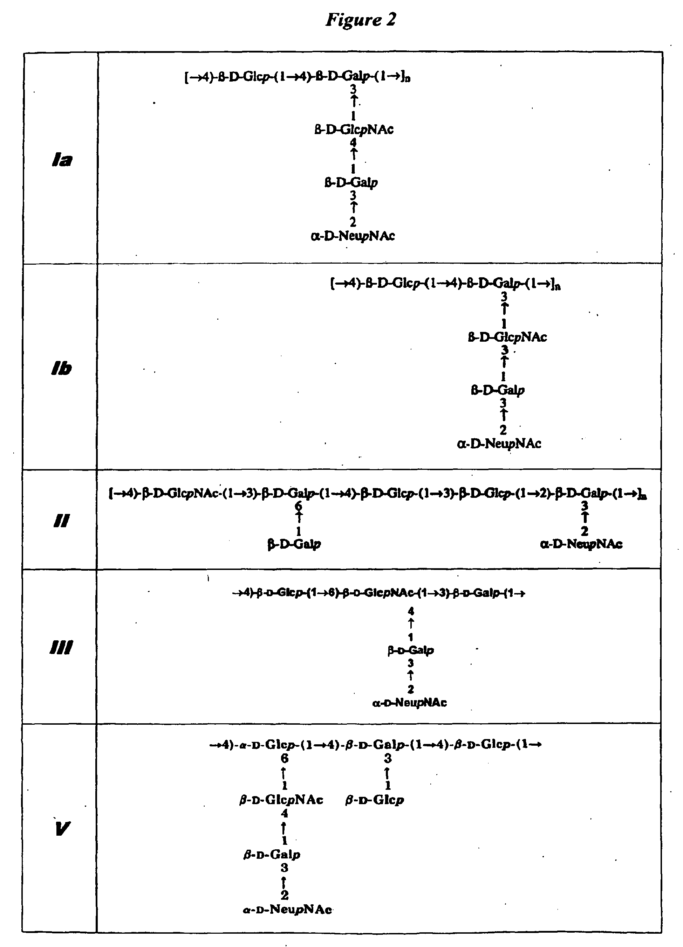

GBS-related disease arises primarily from serotypes Ia, Ib, II, III, IV, V, VI, VII, and VIII, with over 90% being caused by five serotypes: Ia, Ib, II, III & V. The sample preferably comprises a saccharide from one of these five serotypes. As shown in Figure 2, the capsular saccharides of each of these five serotypes include: (a) a terminal N-acetyl-neuraminic acid (NeuNAc) residue (commonly referred to as sialic acid), which in all cases is linked 2→3 to a galactose residue; and (b) a *N*-acetylglucosamine residue (GlcNAc) within the trisaccharide core.

All five saccharides include galactose residues within the trisaccharide core, but serotypes Ia, Ib, II & III also contain additional galactose residues in each repeating unit, with the serotype II saccharide containing three galactose residues per repeating unit.

It is particularly preferred that the sample comprises a GBS saccharide of serotypes Ia, Ib or III.

Preferred conjugate saccharides are saccharides containing a sialic acid residue, preferably a terminal sialic acid residue (*e.g.* GBS capsular saccharide, *N.meningitidis* serogroup B saccharide, *N.meningitidis* serogroup C saccharide, *N.meningitidis* serogroup W135 saccharide, *N.meningitidis* serogroup Y saccharide, *E. coli* K 1 saccharide, and some saccharides of *Haemophilus* strains).

In addition to being useful for analysing full-length capsular saccharides, the invention can be used with oligosaccharide fragments of them.

Other saccharides in conjugates can include glucans (*e.g.* fungal glucans, such as those in *Candida albicans*), and fungal capsular saccharides *e.g.* from the capsule of *Cryptococcus neoformans*. Other preferred conjugate saccharide antigens are eukaryotic saccharides *e.g.* fungal saccharides, plant saccharides, human saccharides (*e.g.* cancer antigens), *etc.* Other conjugate saccharides are lipopolysaccharides and lipooligosaccharides.

Conjugate saccharides that are charged (*e.g.* anionic) at neutral pH are preferred. Saccharides with multiple phosphate and/or multiple carboxylate groups can be analysed using the methods of the invention. The invention is thus particularly useful for analysing samples comprising polyanionic saccharides.

Vaccines

Preferred samples used in the present invention are vaccines comprising conjugated saccharide.

Preferred conjugate vaccines comprise immunogens protecting against:

- *Haemophilus influenzae* type b (Hib);

- *Neisseria meningitidis* (meningococcus) of serogroups A, C W135 and/or Y;

- *Streptococcus pneumoniae* (pneumococcus) of serotypes 4, 6B, 9V, 14, 18C, 19F or 23F;

- *Streptococcus agalactiae* (group B streptococcus) of serotypes Ia, Ib, II, III, IV, V, VI, VII, and/or, especially of serotypes Ia, Ib, II, III and/or V;

- *Pseudomonas aeruginosa;* or

- *Staphylococcus aureus,*

either singly or in combination.

Particularly preferred conjugate vaccines comprise immunogens protecting against *Streptococcus agalactiae* (group B streptococcus).

Preferred combination conjugate vaccines comprise:

- mixtures of conjugates from each of meningococcal serogroups C and Y;

- mixtures of conjugates from each of meningococcal serogroups C, W135 and Y;

- mixtures of conjugates from each of meningococcal serogroups A, C, W135 and Y;

- mixtures of conjugates from meningococcal serogroups A and C;

- mixtures of pneumoccal conjugates;

- mixed meningococcal and Hib conjugates (e.g. mixtures of Hib conjugates and conjugates from each of meningococcal serogroups A and C, or mixtures of Hib conjugates and conjugates from each of meningococcal serogroups C and Y); or

- combined meningococcal, pneumococcal and Hib conjugates.

In addition to the conjugate, the vaccine may contain one or more of:

- a protein antigen from serogroup B of *N.meningitidis;*

- preparations of vesicles prepared from *N.meningitidis* serogroup B;
- an antigen from hepatitis A virus, such as inactivated virus [*e.g.* 45,46];
- an antigen from hepatitis B virus, such as the surface and/or core antigens [*e.g.* 46,47];
- an antigen from *Bordetella pertussis,* such as pertussis holotoxin (PT) and filamentous haemagglutinin (FHA) from *B.pertussis,* optionally also in combination with pertactin and/or agglutinogens 2 and 3. Cellular pertussis antigens may be used instead;
- a diphtheria antigen, such as a diphtheria toxoid [*e.g.* chapter 13 of ref. 1];
- a tetanus antigen, such as a tetanus toxoid [*e.g.* chapter 27 of ref. 1]; or
- polio antigen(s), *e.g.* IPV.

Such antigens may be adsorbed to an aluminium salt adjuvant (*e.g.* a hydroxide or a phosphate). Any further saccharide antigens are preferably included as conjugates.

***Use of the Invention in the Production and Quality Control of Vaccines***

The invention also provides a method of monitoring the stability of a vaccine in storage, comprising the steps of: (a) analysing the vaccine as described herein; and, if the results from step (a) indicate that the vaccine is acceptable for clinical use, *e.g.* it is of suitable unconjugated saccharide or carrier content, (b) either (i) continuing to store the vaccine or (ii) releasing the vaccine for use by physicians. Step (a) may be performed on a packaged vaccine, on a bulk vaccine prior to packaging, on saccharides prior to conjugation; *etc.*

The method of analysis of the invention also allows the comparison of the same vaccine under different conditions, or different vaccines under the same conditions.

Thus, the invention provides a method of comparing different vaccines, comprising the steps of: (a) treating a plurality of different vaccines under substantially identical environmental conditions; (b) analysing the treated vaccines as described herein; (c) comparing the results of step (b); and, optionally, (d) selecting a vaccine, *e.g.* a vaccine stable under the at least one environmental condition from the plurality of different vaccines. Step (d) may, for example, comprise selecting the most stable vaccine under the at least one environmental condition. Thus, uses for this method include comparing the stability of different vaccines, *e.g.* under storage conditions. The environmental condition can be a chemical condition (*e.g.* exposure to a chemical component, *e.g.* a solvent, carrier *etc.*), pH, temperature, humidity *etc.* or a combination thereof. The plurality of different vaccines can typically differ in their composition, *e.g.* length of the saccharide, linker between the saccharide and the carrier, the carrier, presence of other vaccine components, concentration of components, excipients, adjuvants, pH, osmolarity, ionic strength *etc.*

The invention also provides a method of comparing the effect of different environmental conditions on a vaccine, comprising the steps of: (a) treating a plurality of substantially identical samples of a vaccine under a plurality of different environmental conditions; (b) analysing the treated samples as described herein; and (c) comparing the results of step (b); and, optionally, (d) selecting an environmental condition, *e.g.* an environmental condition under which the vaccine is stable from the plurality of different environmental conditions. Step (d) may, for example, comprise selecting the environmental condition under which the vaccine is most stable. Uses for this method include optimising the storage conditions of a vaccine. The environmental condition can be a chemical condition (*e.g.* exposure to a chemical component, *e.g.* a solvent, carrier *etc.*), pH, temperature, humidity *etc.* or a combination thereof.

***General***

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value $x$ means, for example, $x \pm 10\%$.

**BRIEF DESCRIPTION OF DRAWINGS**

Figure 1 shows the structures of, and differences between, the capsular saccharides of GBS serotypes Ia and III.

Figure 2 shows the structures of the capsular saccharides of GBS serotypes Ia, Ib, II, III and V.

Figure 3 (plots A and B shows the results of a colorimetric assay with and without $K_2HPO_4$ and shows that $K_2HPO_4$ does not interefere with saccharide analysis by colorimetric assay.

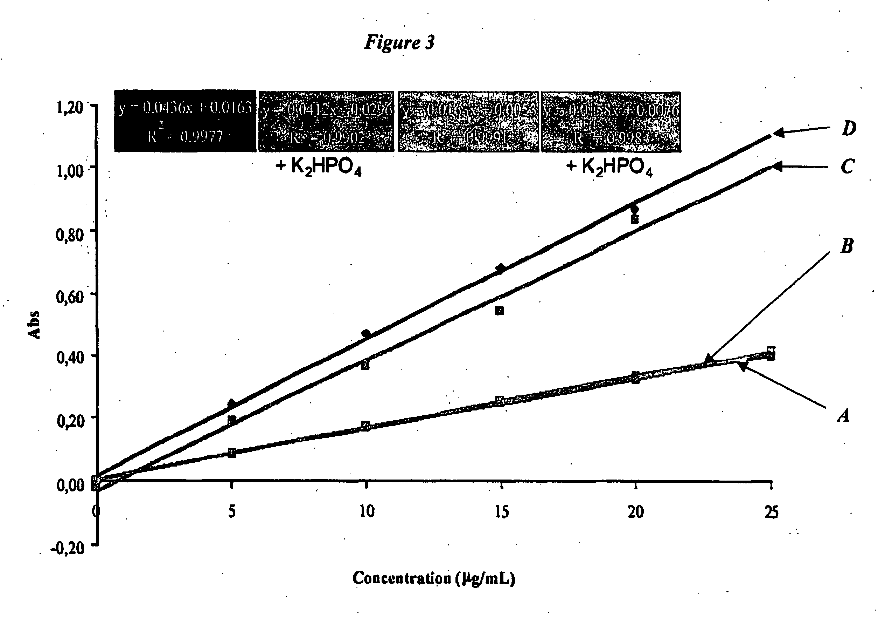

Figure 3 (plots C and D) shows the results of a micro BCA assay with and without $K_2HPO_4$ and shows that $K_2HPO_4$ does not interefere with carrier analysis by micro BCA assay.

## MODES FOR CARRYING OUT THE INVENTION

**Quantification of low level unconjugated saccharide in TT, $CRM_{197}$ GBS67 or GBS80 conjugate vaccine following separation by selective precipitation**

The invention is exemplified by the analysis of the unconjugated saccharide content of GBS conjugate vaccines of TT (tetanus toxoid), $CRM_{197}$ (diphtheria toxin derivative), GBS67 or GBS80. The conjugate vaccines were buffered in 10 mM sodium phosphate solution at pH 7.2.

*1. Separation of conjugates by salt precipitation*

In order to precipitate the conjugates, 500 $\mu$L $K_2HPO_4$ saturated solution (150 g per 100 mL of cold $H_2O$ (-8.6 M; pH 10.30)) was added to 500 $\mu$L of bulk conjugate (saccharide content ~1 mg/mL; protein content -0.8 mg/mL) resulting in a mixture having a pH of 9.66. The mixture was incubated in 0°C ice bath for 10 min.
The mixture was centrifugated in a Beckman Microfuge 11 at 13000 rpm for 20 min and the pellet adsorbed to the wall of the vial. The supernatant was removed and then analysed to estimate the saccharide content.

*2. Determination of sialic acid content by colorimetric assay*

The sialic acid content in the bulk conjugate (total polysaccharide as free and bound polysaccharide) and in the precipitation supernatant (free polysaccharide) was estimated by the Svennerholm method [94] (modified by heating at 110°C for 20 minutes instead of 40 minutes).
The percentage unconjugated polysaccharide (PS) content was calculated by the ratio:

$$\text{\% unconjugated PS} = [\text{free PS / total PS}] * 100$$

*3. Results*

The percentage unconjugated (free) saccharide of the various conjugate vaccines were determined as follows (table 1):

Table 1

| GBS type | Carrier | % Free Saccharide |
|---|---|---|
| Ia | CRM | <1.0% |
| | GBS80 | 3.5% |
| | GBS67 | <1& |
| Ib | CRM | 1.8% |
| | GBS80 | 14.8% |
| | GBS67 | < 1.0% |
| III | CRM | 1.6% |
| | CRM | 4.4% |
| | TetTox | 3.8% |
| | GBS80 | 9.1% |
| | GBS67 | <1.0% |

The invention thus provides a simple and rapid precipitation method for separating low levels of free saccharide from conjugates in a bulk product. The unconjugated saccharide was found to stay in the supernatant without precipitation, while conjugated saccharide is precipitated.

### The basic reagent does not interfere with saccharide analysis

To ensure that the $K_2HPO_4$ does not interfere with the colorimetric assay, standard solutions of sialic acid, with and without addition of $K_2HPO_4$ solution, were assayed.

As can be seen from figure 3, the results of the colorimetric assay with addition of $K_2HPO_4$ (plot A) are almost identical to the results without $K_2HPO_4$ (plot B). The absolute values of the colorimetric assay are shown on the *y*-axis as a function of sialic acid concentration. It can be seen that precipitation with $K_2HPO_4$ in the methods of the invention does not interfere with the colorimetric assay.

### The basic reagent does not interfere with carrier analysis

To ensure that the $K_2HPO_4$ does not interfere with carrier analysis, standard solutions of bovine serum albumin (BSA) as a test carrier, with and without addition of $K_2HPO_4$ solution, were assayed by a micro BCA assay.

As can be seen from figure 3, the results of the micro BCA assay with addition of $K_2HPO_4$ (plot C) are very similar to the results without $K_2HPO_4$ (plot D). The absolute values of the BCA assay are shown on the *y*-axis as a function of BSA concentration. It can be seen that precipitation with $K_2HPO_4$ in the methods of the invention does not interfere with the BCA assay.

It will be understood that the invention has been described by way of example only and modifications may be made whilst remaining within the scope of the invention, which is defined in the appened claims.

## REFERENCES

[1] Vaccines (eds. Plotkin et al.) 4th edition, ISBN: 0721696880.

[2] Baker et al. (2003) J Infect Dis 188:66-73.

[3] Theilacker et al. (2003) Infect Immun 71:3875-84.

[4] Anonymous (2003) Drugs R D 4:383-5.

[5] Jones (2001) Curr Opin Investig Drugs 2:47-49.

[6] WO02/058737

[7] WO03/007985.

[8] Rennels et al. (2002) Pediatr Infect Dis J 21:978-979.

[9] Campbell et al. (2002) J Infect Dis 186:1848-1851.

[10] Costantino et al. (1992) Vaccine 10:691-698.

[11] Lieberman et al. (1996) JAMA 275:1499-1503.

[12] Darkes & Plosker (2002) Paediatr Drugs 4:609-630.

[13] Ugozzoli (2002) J Infect Dis 186:1358-61.

[14] Granoff et al. (1997) Infect Immun 65:1710-5.

[15] Paradiso & Lindberg (1996) Dev Biol Stand 87:269-275.

[16] Corbel (1996) Dev Biol Stand 87:113-124.

[17] WO03/080678.

[18] Klug (1996) Dev Biol Stand 87:263-267.

[19] Plumb & Yost (1996) Vaccine 14:399-404.

[20] Lei et al. (2000) Dev Biol (Basel) 103:259-64.

[21] Jumel et al. (2002) Biotechnol Appl Biochem 36:219-226.

[22] Bardotti et al. (2000) Vaccine 18:1982-1993

[23] Deng et al. (2000) J. Biol Chem 275(11):7497-7504

[24] Paoletti et al. (1990) J Biol Chem 265:18278-83

[25] Paoletti et al. (1992) J Clin Invest 89:203-9

[26] Wessels et al. (1987) Proc Natl Acad Sci USA 84:9170-4

[27] Wang et al. (2003) Vaccine 21: 1112-7

[28] US patents 6027733 & 6274144

[29] WO2005/090986.

[30] Hardy et al. (1988) Anal Biochem 170:54-62.

[31] Wang et al. (1990) Anal Biochem 190:182-187.

[32] Ashwell (1957) Methods Enzymology 3:73-105

[33] Stoscheck (1990) Methods Enzymology 182:50-68

[34] WO02/09643.

[35] Katial et al. (2002) Infect Immun 70:702-707.

[36] WO01/52885.

[37] European patent 0301992.

[38] Bjune et al. (1991) Lancet 338(8775):1093-1096.

[39] Fukasawa et al. (1999) Vaccine 17:2951-2958.

[40] WO02/09746.

[41] Rosenqvist et al. (1998) Dev. Biol. Stand. 92:323-333.

[42] Watson (2000) Pediatr Infect Dis J 19:331-332.

[43] Rubin (2000) Pediatr Clin North Am 47:269-285, v.

[44] Jedrzejas (2001) Microbiol Mol Biol Rev 65:187-207.

[45] Bell (2000) Pediatr Infect Dis J 19:1187-1188.

[46] Iwarson (1995) APMIS 103:321-326.

[47] Gerlich et al. (1990) Vaccine 8 Suppl:S63-68 & 79-80.

[48] Gustafsson et al. (1996) N. Engl. J. Med. 334:349-355.

[49] Rappuoli et al. (1991) TIBTECH 9:232-238.

[50] Del Guidice et al. (1998) Molecular Aspects of Medicine 19:1-70.

[51] Sutter et al. (2000) Pediatr Clin North Am 47:287-308.

[52] Zimmenman & Spann (1999) Am Fam Physician 59:113-118, 125-126.

[53] Ramsay et al. (2001) Lancet 357(9251):195-196.

[54] Lindberg (1999) Vaccine 17 Suppl 2:S28-36.

[55] Buttery & Moxon (2000) JR Coll Physicians Lond 34:163-168.

[56] Ahmad & Chapnick (1999) Infect Dis Clin North Am 13:113-133, vii.

[57] Goldblatt (1998) J. Med. Microbiol. 47:563-567.

[58] European patent 0477508.

[59] US patent 5,306,492.

[60] WO98/42721.

[61] Conjugate Vaccines (eds. Cruse et al.) ISBN 3805549326, particularly vol. 10:48-114.

[62] Hermanson (1996) Bioconjugate Techniques ISBN: 0123423368 or 012342335X.

[63] US patent 4,761,283

[64] US patent 4,356,170

[65] WO00/10599

[66] Gever et al. Med. Microbiol. Immunol, 165 : 171-288 (1979).

[67] US patent 4,057,685.

[68] US patents 4,673,574; 4,761,283; 4,808,700.

[69] US patent 4,459,286.

[70] US patent 4,965,338

[71] US patent 4,663,160.

[72] Anonymous (Jan 2002) Research Disclosure, 453077.

[73] Anderson (1983) Infect Immun 39(1):233-238.

[74] Anderson et al. (1985) J Clin Invest 76(1):52-59.

[75] EP-A-0372501.

[76] EP-A-0378881.

[77] EP-A-0427347.

[78] WO93/17712

[79] WO94/03208.

[80] WO98/58668.

[81] EP-A-0471177.

[82] WO91/01146

[83] Falugi et al. (2001) Eur J Immunol 31:3816-3824.

[84] Baraldo et al, (2004) Infect Immun.72:4884-7

[85] EP-A-0594610.

[86] WO00/56360.

[87] WO02/091998.

[88] WO01/72337

[89] WO00/61761.

[90] WO99/42130

[91] Glode et al. (1979) J Infect Dis 139:52-56

[92] WO94/05325; US patent 5,425,946.

[93] WO2005/033148

[94] Svennerholm (1957) Biochem Biophys Acta 24:604-11

**Claims**

1. A method of analysing the degree of unconjugation of a sample comprising a conjugated saccharide component and an unconjugated component, comprising the steps of (i) contacting the sample with a basic reagent under basic conditions to selectively precipitate the conjugated saccharide component from the sample and thereby to obtain a supernatant comprising the separated unconjugated component and (ii) analysing the supernatant's content to give the unconjugated content of the sample, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11.

2. A method of preparing a sample comprising a conjugated saccharide component and an unconjugated component for analysis of its degree of unconjugation, comprising the step of contacting the sample with a basic reagent under basic conditions to selectively precipitate the conjugated saccharide component from the sample and thereby to obtain a supernatant comprising separated unconjugated component, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11.

3. The method of claim 1 or claim 2 wherein the unconjugated component is an unconjugated saccharide component and/or the method further comprises the step of measuring the sample's total saccharide content.

4. The method of claim 1 or claim 2 wherein the unconjugated component is an unconjugated carrier component and/or the method further comprises the step of measuring the sample's total carrier content.

5. A method of separating a conjugated saccharide component in a sample from an unconjugated component in the sample, comprising the step of contacting the sample with a basic reagent under basic conditions to selectively precipitate the conjugated saccharide component from the sample, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11.

6. The use of a basic reagent under basic conditions for selectively precipitating a conjugated saccharide component in a sample from an unconjugated component in the sample, thereby separating the conjugated saccharide component from the unconjugated component, wherein the conjugated saccharide component is a covalently linked saccharide-carrier conjugate, the conjugated saccharide is selectively precipitated from the unconjugated saccharide with the basic reagent under basic conditions, and the basic conditions are from pH 9 to 11.

7. The method of claim 5 or use of claim 6 wherein the unconjugated component is an unconjugated saccharide component or an unconjugated carrier component.

8. The method of any one of claims 1 to 5 or 7 or the use of any of claims 6 to 7 wherein the basic reagent comprises a lyotropic salt that is $K_2HPO_4$.

9. The method of any of claims 1 to 5 or 7 to 8 or the use of any of claims 6 to 8, wherein the basic conditions are from pH 9.5 to 9.9 and optionally the basic reagent comprises $K_2HPO_4$.

10. The method of any of claims 1 to 5 or 7 to 9 or the use of any of claims 6 to 9 wherein the conjugated saccharide is a saccharide antigen conjugated to a carrier protein.

11. The method of any of claims 1 to 5 or 7 to 10 or the use of any of claims 6 to 10 wherein the sample is a vaccine, particularly a glycoconjugate vaccine, more particularly a glycoconjugate vaccine comprising a conjugate comprising a saccharide containing a sialic acid residue or a glycoconjugate vaccine comprising a conjugate comprising a bacterial capsular saccharide from *Streptococcus agalactiae, e.g.* from *Streptococcus agalactiae* serogroup Ia, Ib, II, III or V.

12. A method of releasing a vaccine for use by physicians, comprising the steps of: (a) manufacturing a vaccine comprising a conjugated saccharide; (b) analysing the vaccine's degree of unconjugation by a method of claim 1 or claim 2; and, if the results from step (b) indicate a degree of unconjugation acceptable for clinical use, (c) releasing the vaccine for use by physicians.

13. A method for preparing a vaccine composition, comprising a step of analysing the vaccine's degree of unconjugation by a method of claim 1 or claim 2, including a step of pH measurement, followed by a step of adjusting the pH of the composition to a desired value *e.g.* between 6 and 8, or about 7.

14. A method for packaging a vaccine, comprising the steps of: (a) manufacturing a bulk vaccine containing a conjugated saccharide; (b) analysing the degree of unconjugation of the bulk vaccine by a method of claim 1 or claim 2; (c) optionally, analysing the bulk vaccine for pH and/or other properties; and, if the results from step (b) and (c) indicate that the bulk vaccine is acceptable for clinical use, (d) preparing and packaging the vaccine for human use from the bulk.

**Patentansprüche**

1. Verfahren zur Analyse des Grads der Nichtkonjugation einer eine konjugierte Saccharidkomponente und eine nichtkonjugierte Komponente umfassenden Probe, mit den folgenden Schritten: (i) Inkontaktbringen der Probe mit einem basischen Reagens unter basischen Bedingungen zum selektiven Fällen der konjugierten Saccharidkomponente aus der Probe und damit Gewinnen eines die getrennte nichtkonjugierte Komponente umfassenden Überstands und (ii) Analysieren des Gehalts des Überstands, woraus sich der nichtkonjugierte Gehalt der Probe ergibt, wobei es sich bei der konjugierten Saccharidkomponente um ein kovalent verknüpftes Saccharid-Träger-Konjugat handelt, das konjugierte Saccharid aus dem nichtkonjugierten Saccharid mit dem basischen Reagens unter basischen Bedingungen selektiv gefällt wird und es sich bei den basischen Bedingungen um einen pH-Wert von 9 bis 11 handelt.

2. Verfahren zur Herstellung einer eine konjugierte Saccharidkomponente und eine nichtkonjugierte Komponente umfassenden Probe zur Analyse ihres Grads der Nichtkonjugation, mit dem Schritt des Inkontaktbringens der Probe mit einem basischen Reagens unter basischen Bedingungen zum selektiven Fällen der konjugierten Saccharidkomponente aus der Probe und damit Gewinnen eines getrennte nichtkonjugierte Komponente umfassenden Überstands, wobei es sich bei der konjugierten Saccharidkomponente um ein kovalent verknüpftes Saccharid-Träger-Konjugat handelt, das konjugierte Saccharid aus dem nichtkonjugierten Saccharid mit dem basischen Reagens unter basischen Bedingungen selektiv gefällt wird und es sich bei den basischen Bedingungen um einen pH-Wert von 9 bis 11 handelt.

3. Verfahren nach Anspruch 1 oder Anspruch 2, wobei es sich bei der nichtkonjugierten Komponente um eine nichtkonjugierte Saccharidkomponente handelt und/oder das Verfahren ferner den Schritt des Messens des Gesamtsaccharidgehalts der Probe umfasst.

4. Verfahren nach Anspruch 1 oder Anspruch 2, wobei es sich bei der nichtkonjugierten Komponente um eine nichtkonjugierte Trägerkomponente handelt und/oder das Verfahren ferner den Schritt des Messens des Gesamtträgergehalts der Probe umfasst.

5. Verfahren zur Trennung einer konjugierten Saccharidkomponente in einer Probe von einer nichtkonjugierten Komponente in der Probe, mit dem Schritt des Inkontaktbringens der Probe mit einem basischen Reagens unter basischen Bedingungen zum selektiven Fällen der konjugierten Saccharidkomponente aus der Probe, wobei es sich bei der konjugierten Saccharidkomponente um ein kovalent verknüpftes Saccharid-Träger-Konjugat handelt, das konjugierte Saccharid aus dem nichtkonjugierten Saccharid mit dem basischen Reagens unter basischen Bedingungen selektiv gefällt wird und es sich bei den basischen Bedingungen um einen pH-Wert von 9 bis 11 handelt.

6. Verwendung eines basischen Reagens unter basischen Bedingungen zum selektiven Fällen einer konjugierten Saccharidkomponente in einer Probe aus einer nichtkonjugierten Komponente in der Probe und damit Trennen der konjugierten Saccharidkomponente von der nichtkonjugierten Komponente, wobei es sich bei der konjugierten Saccharidkomponente um ein kovalent verknüpftes Saccharid-Träger-Konjugat handelt, das konjugierte Saccharid aus dem nichtkonjugierten Saccharid mit dem basischen Reagens unter basischen Bedingungen selektiv gefällt wird und es sich bei den basischen Bedingungen um einen pH-Wert von 9 bis 11 handelt.

7. Verfahren nach Anspruch 5 oder Verwendung nach Anspruch 6, wobei es sich bei der nichtkonjugierten Komponente um eine nichtkonjugierte Saccharidkomponente oder eine nichtkonjugierte Trägerkomponente handelt.

8. Verfahren nach einem der Ansprüche 1 bis 5 oder 7 oder Verwendung nach einem der Ansprüche 6 bis 7, wobei das basische Reagens ein lyotropes Salz umfasst, bei dem es sich um $K_2HPO_4$ handelt.

9. Verfahren nach einem der Ansprüche 1 bis 5 oder 7 bis 8 oder Verwendung nach einem der Ansprüche 6 bis 8, wobei es sich bei den basischen Bedingungen um einen pH-Wert von 9,5 bis 9,9 handelt und das basische Reagens gegebenenfalls $K_2HPO_4$ umfasst.

10. Verfahren nach einem der Ansprüche 1 bis 5 oder 7 bis 9 oder Verwendung nach einem der Ansprüche 6 bis 9, wobei es sich bei dem konjugierten Saccharid um ein an ein Trägerprotein konjugiertes Saccharidantigen handelt.

11. Verfahren nach einem der Ansprüche 1 bis 5 oder 7 bis 10 oder Verwendung nach einem der Ansprüche 6 bis 10, wobei es sich bei der Probe um einen Impfstoff, insbesondere einen Glykokonjugat-Impfstoff, ganz besonders einen ein ein Saccharid mit einem Sialinsäurerest umfassendes Konjugat umfassenden Glykokonjugat-Impfstoff oder einen ein ein bakterielles Kapselsaccharid aus *Streptococcus agalactiae,* z.B. aus *Streptococcus agalactiae* Serogruppe Ia, Ib, II, III oder V umfassendes Konjugat umfassenden Glykokonjugat-Impfstoff handelt.

12. Verfahren zum Freigeben eines Impfstoffs zum ärztlichen Gebrauch, mit den folgenden Schritten: (a) Herstellen eines ein konjugiertes Saccharid umfassenden Impfstoffs; (b) Analysieren des Grads der Nichtkonjugation des Impfstoffs mit einem Verfahren nach Anspruch 1 oder Anspruch 2; und, falls die Ergebnisse aus Schritt (b) einen für die klinische Anwendung unbedenklichen Grad der Nichtkonjugation anzeigen, (c) Freigeben des Impfstoffs zum ärztlichen Gebrauch.

13. Verfahren zur Herstellung einer Impfstoffzusammensetzung, mit einem Schritt zum Analysieren des Grads der Nichtkonjugation des Impfstoffs mit einem Verfahren nach Anspruch 1 oder Anspruch 2, einschließlich eines Schritts zur pH-Messung, und einem anschließenden Schritt zum Einstellen des pH-Werts der Zusammensetzung auf einen gewünschten Wert, z.B. zwischen 6 und 8 oder um 7.

14. Verfahren zum Abpacken eines Impfstoffs, mit den folgenden Schritten: (a) Herstellen einer Großmenge Impfstoff mit einem konjugierten Saccharid; (b) Analysieren des Grads der Nichtkonjugation der Großmenge Impfstoff mit einem Verfahren nach Anspruch 1 oder Anspruch 2; (c) gegebenenfalls Analysieren der Großmenge Impfstoff auf pH-Wert und/oder andere Eigenschaften; und, falls die Ergebnisse aus Schritt (b) und (c) anzeigen, dass die Großmenge Impfstoff für die klinische Anwendung unbedenklich ist, (d) Herstellen und Abpacken des Impfstoffs aus der Großmenge zur Anwendung beim Menschen.

**Revendications**

1. Procédé d'analyse du degré de non conjugaison d'un échantillon comprenant un composant saccharide conjugué et un composant non conjugué, comprenant les étapes de (i) mise en contact de l'échantillon avec un réactif basique dans des conditions basiques pour provoquer la précipitation sélective du composant saccharide conjugué à partir de l'échantillon, de façon à obtenir de ce fait un surnageant comprenant le composant non conjugué séparé, et (ii) analyse du contenu du surnageant, pour donner le contenu non conjugué de l'échantillon, le composant saccharide conjugué étant un conjugué saccharide-support lié par liaison covalente, le saccharide conjugué étant soumis à une précipitation sélective, à partir du saccharide non conjugué, avec le réactif basique dans des conditions basiques, et les conditions basiques correspondant à un pH de 9 à 11.

2. Procédé de préparation d'un échantillon comprenant un composant saccharide conjugué et un composant non conjugué pour analyse de son degré de non conjugaison, comprenant l'étape de mise en contact de l'échantillon avec un réactif basique dans des conditions basiques pour provoquer la précipitation sélective du composant saccharide conjugué à partir de l'échantillon et obtenir de ce fait un surnageant comprenant le composant non conjugué séparé, le composé saccharide conjugué étant un conjugué saccharide-support lié par liaison covalente, le saccharide conjugué étant soumis à une précipitation sélective à partir du saccharide non conjugué avec le réactif basique dans des conditions basiques, et les conditions basiques correspondant à un pH de 9 à 11.

3. Procédé de la revendication 1 ou 2, dans lequel le composant non conjugué est un composant saccharide non conjugué, et/ou le procédé comprend en outre l'étape de mesure de la teneur totale de l'échantillon en saccharide.

4. Procédé de la revendication 1 ou 2, dans lequel le composant non conjugué est un composant support non conjugué, et/ou le procédé comprend en outre l'étape de mesure de la teneur totale de l'échantillon en support.

5. Procédé de séparation d'un composant saccharide conjugué se trouvant dans un échantillon, d'avec un composant non conjugué se trouvant dans l'échantillon, comprenant l'étape de mise en contact de l'échantillon avec un réactif basique dans des conditions basiques pour provoquer la précipitation sélective du composant saccharide conjugué à partir de l'échantillon, le composant saccharide conjugué étant un conjugué saccharide-support lié par liaison covalente, le saccharide conjugué étant soumis à une précipitation sélective à partir du saccharide non conjugué avec le réactif basique dans des conditions basiques, et les conditions basiques correspondant à un pH de 9 à 11.

6. Utilisation d'un réactif basique dans des conditions basiques pour provoquer la précipitation sélective d'un composant saccharide conjugué se trouvant dans un échantillon, à partir d'un composant non conjugué se trouvant dans l'échantillon, de façon à séparer le composant saccharide conjugué d'avec le composant non conjugué, le composant saccharide conjugué étant un conjugué saccharide-support lié par liaison covalente, le saccharide conjugué étant soumis à une précipitation sélective à partir du saccharide non conjugué avec le réactif basique dans des conditions basiques, et les conditions basiques correspondant à un pH de 9 à 11.

7. Procédé de la revendication 5 ou utilisation de la revendication 6, le composant non conjugué étant un composant saccharide non conjugué ou un composant support non conjugué.

8. Procédé de l'une quelconque des revendications 1 à 5 ou 7 ou utilisation de l'une quelconque des revendications 6 et 7, le réactif basique comprenant un sel lyotrope, qui est $K_2HPO_4$.

9. Procédé de l'une quelconque des revendications 1 à 5 ou 7 et 8, ou utilisation de l'une quelconque des revendications 6 à 8, les conditions basiques correspondant à un pH de 9,5 à 9,9, et en option le réactif basique comprenant du $K_2HPO_4$.

10. Procédé de l'une quelconque des revendications 1 à 5 ou 7 à 9 ou utilisation de l'une quelconque des revendications 6 à 9, le saccharide conjugué étant un antigène saccharidique conjugué à une protéine support.

11. Procédé de l'une quelconque des revendications 1 à 5 ou 7 à 10 ou utilisation de l'une quelconque des revendications 6 à 10, l'échantillon étant un vaccin, en particulier un vaccin glycoconjugué, plus particulièrement un vaccin glycoconjugué comprenant un conjugué comprenant un saccharide contenant un résidu d'acide sialique ou un vaccin glycoconjugué comprenant un conjugué comprenant un saccharide de capsule bactérienne de *Streptococcus agalactiae,* par exemple de *Streptococcus agalactiae* sérogroupe Ia, Ib, II, III ou V.

12. Procédé de libération d'un vaccin pour utilisation par des médecins, comprenant les étapes de (a) fabrication d'un vaccin comprenant un saccharide conjugué ; (b) analyse du degré de non conjugaison du vaccin par un procédé de la revendication 1 ou 2 ; et, si les résultats de l'étape (b) indiquent un degré de non conjugaison acceptable pour utilisation clinique, (c) libération du vaccin pour utilisation par des médecins.

13. Procédé de préparation d'une composition vaccinale, comprenant une étape d'analyse du degré de non conjugaison du vaccin par un procédé de la revendication 1 ou 2, y compris une étape de mesure du pH, suivie d'une étape d'ajustement du pH de la composition à une valeur souhaitée, par exemple entre 6 et 8, ou d'environ 7.

14. Procédé de conditionnement d'un vaccin, comprenant les étapes de : (a) fabrication d'un vaccin en vrac contenant un saccharide conjugué ; (b) analyse du degré de non conjugaison du vaccin en vrac par un procédé de la revendication 1 ou 2 ; (c) en option, analyse du pH et/ou d'autres propriétés du vaccin en vrac ; et, si les résultats des étapes (b) et (c) indiquent que le vaccin en vrac est acceptable pour utilisation clinique, (d) préparation et conditionnement du vaccin, à partir du vrac, pour utilisation humaine.

## Figure 1

**Ia**              **III**           *A*

$$[\boxed{\rightarrow 4}\text{-}\beta\text{-Gal-}(1\rightarrow 4)\text{-}\beta\text{-Glc-}(1\rightarrow ]_n \qquad \beta\text{-Gal-}(1\rightarrow 4)\text{-}\beta\text{-Glc-}(1\rightarrow ]_n$$

$$3 \qquad\qquad\qquad\qquad\qquad\qquad 3$$
$$\uparrow \qquad\qquad\qquad\qquad\qquad\qquad \uparrow$$
$$1 \qquad\qquad\qquad\qquad\qquad\qquad 1$$

β-GlcNAc          $[\boxed{\rightarrow 6}\text{-}\beta$-GlcNAc     *B*

$$4 \qquad\qquad\qquad\qquad\qquad\qquad 4$$
$$\uparrow \qquad\qquad\qquad\qquad\qquad\qquad \uparrow$$
$$1 \qquad\qquad\qquad\qquad\qquad\qquad 1$$

β-Gal                  β-Gal

$$3 \qquad\qquad\qquad\qquad\qquad\qquad 3$$
$$\uparrow \qquad\qquad\qquad\qquad\qquad\qquad \uparrow \qquad\qquad\quad C$$
$$2 \qquad\qquad\qquad\qquad\qquad\qquad 2$$

α-NeuNAc          α-NeuNAc

## Figure 2

*Figure 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02058737 A
- WO 03007985 A
- WO 03080678 A
- US 6027733 A
- US 6274144 A
- WO 2005090986 A
- WO 0209643 A
- WO 0152885 A
- EP 0301992 A
- WO 0209746 A
- EP 0477508 A
- US 5306492 A
- WO 9842721 A
- US 4761283 A
- US 4356170 A
- WO 0010599 A
- US 4057685 A
- US 4673574 A
- US 4808700 A
- US 4459286 A
- US 4965338 A
- US 4663160 A
- EP 0372501 A
- EP 0378881 A
- EP 0427347 A
- WO 9317712 A
- WO 9403208 A
- WO 9858668 A
- EP 0471177 A
- WO 9101146 A
- EP 0594610 A
- WO 0056360 A
- WO 02091998 A
- WO 0172337 A
- WO 0061761 A
- WO 9942130 A
- WO 9405325 A
- US 5425946 A
- WO 2005033148 A

### Non-patent literature cited in the description

- Vaccines
- **BAKER et al.** *J Infect Dis,* 2003, vol. 188, 66-73
- **THEILACKER et al.** *Infect Immun,* 2003, vol. 71, 3875-84
- **ANONYMOUS.** *Drugs R D,* 2003, vol. 4, 383-5
- **JONES.** *Curr Opin Investig Drugs,* 2001, vol. 2, 47-49
- **RENNELS et al.** *Pediatr Infect Dis J,* 2002, vol. 21, 978-979
- **CAMPBELL et al.** *J Infect Dis,* 2002, vol. 186, 1848-1851
- **COSTANTINO et al.** *Vaccine,* 1992, vol. 10, 691-698
- **LIEBERMAN et al.** *JAMA,* 1996, vol. 275, 1499-1503
- **DARKES ; PLOSKER.** *Paediatr Drugs,* 2002, vol. 4, 609-630
- **UGOZZOLI.** *J Infect Dis,* 2002, vol. 186, 1358-61
- **GRANOFF et al.** *Infect Immun,* 1997, vol. 65, 1710-5
- **PARADISO ; LINDBERG.** *Dev Biol Stand,* 1996, vol. 87, 269-275
- **CORBEL.** *Dev Biol Stand,* 1996, vol. 87, 113-124
- **KLUG.** *Dev Biol Stand,* 1996, vol. 87, 263-267
- **PLUMB ; YOST.** *Vaccine,* 1996, vol. 14, 399-404
- **LEI et al.** *Dev Biol (Basel,* 2000, vol. 103, 259-64
- **JUMEL et al.** *Biotechnol Appl Biochem,* 2002, vol. 36, 219-226
- **BARDOTTI et al.** *Vaccine,* 2000, vol. 18, 1982-1993
- **DENG et al.** *J. Biol Chem,* 2000, vol. 275 (11), 7497-7504
- **PAOLETTI et al.** *J Biol Chem,* 1990, vol. 265, 18278-83
- **PAOLETTI et al.** *J Clin Invest,* 1992, vol. 89, 203-9
- **WESSELS et al.** *Proc Natl Acad Sci USA,* 1987, vol. 84, 9170-4
- **WANG et al.** *Vaccine,* 2003, vol. 21, 1112-7
- **HARDY et al.** *Anal Biochem,* 1988, vol. 170, 54-62
- **WANG et al.** *Anal Biochem,* 1990, vol. 190, 182-187

- **ASHWELL.** *Methods Enzymology,* 1957, vol. 3, 73-105
- **STOSCHECK.** *Methods Enzymology,* 1990, vol. 182, 50-68
- **KATIAL et al.** *Infect Immun,* 2002, vol. 70, 702-707
- **BJUNE et al.** *Lancet,* 1991, vol. 338 (8775), 1093-1096
- **FUKASAWA et al.** *Vaccine,* 1999, vol. 17, 2951-2958
- **ROSENQVIST et al.** *Dev. Biol. Stand.,* 1998, vol. 92, 323-333
- **WATSON.** *Pediatr Infect Dis J,* 2000, vol. 19, 331-332
- **RUBIN.** *Pediatr Clin North Am,* 2000, vol. 47, 269-285
- **JEDRZEJAS.** *Microbiol Mol Biol Rev,* 2001, vol. 65, 187-207
- **BELL.** *Pediatr Infect Dis J,* 2001, vol. 19, 1187-1188
- **IWARSON.** *APMIS,* 1995, vol. 103, 321-326
- **GERLICH et al.** *Vaccine,* 1990, vol. 8, 63-6879-80
- **GUSTAFSSON et al.** *N. Engl. J. Med.,* 1996, vol. 334, 349-355
- **RAPPUOLI et al.** *TIBTECH,* 1991, vol. 9, 232-238
- **DEL GUIDICE et al.** *Molecular Aspects of Medicine,* 1998, vol. 19, 1-70
- **SUTTER et al.** *Pediatr Clin North Am,* 2000, vol. 47, 287-308
- **ZIMMENMAN ; SPANN.** *Am Fam Physician,* 1999, vol. 59 (113-118), 125-126
- **RAMSAY et al.** *Lancet,* 2001, vol. 357 (9251), 195-196
- **LINDBERG.** *Vaccine,* 1999, vol. 17 (2), 28-36
- **BUTTERY ; MOXON.** *JR Coll Physicians Lond,* 2000, vol. 34, 163-168
- **AHMAD ; CHAPNICK.** *Infect Dis Clin North Am,* 1999, vol. 13, 113-133
- **GOLDBLATT.** *J. Med. Microbiol.,* 1998, vol. 47, 563-567
- Conjugate Vaccines. vol. 10, 48-114
- **HERMANSON.** *Bioconjugate Techniques,* 1996, ISBN 0123423368 or 012342335X
- **GEVER et al.** *Med. Microbiol. Immunol,* 1979, vol. 165, 171-288
- **ANONYMOUS.** *Research Disclosure,* January 2002, 453077
- **ANDERSON.** *Infect Immun,* 1983, vol. 39 (1), 233-238
- **ANDERSON et al.** *J Clin Invest,* 1985, vol. 76 (1), 52-59
- **FALUGI et al.** *Eur J Immunol,* 2001, vol. 31, 3816-3824
- **BARALDO et al.** *Infect Immun.,* 2004, vol. 72, 4884-7
- **GLODE et al.** *J Infect Dis,* 1979, vol. 139, 52-56
- **SVENNERHOLM.** *Biochem Biophys Acta,* 1957, vol. 24, 604-11